# FASCICULE DE BREVET EUROPEEN

(11) **EP 0 533 579 B1**
(45) Date de publication et mention de la délivrance du brevet: **04.12.1996**
(21) Numéro de dépôt: 92402566.1
(22) Date de dépôt: 18.09.1992
(51) Int. Cl.: C07D 295/08, A61K 31/495, C07D 307/81, C07D 333/58

(54) **Nouveaux dérivés de la trimétazidine, leur procédé de préparation et les compositions pharmaceutiques les contenant**
Trimetazidinederivate, Verfahren zu deren Herstellung und deren pharmazeutischen Zusammensetzungen
Trimetazidine derivatives, process for their preparation and pharmaceutical compositions containing them

(30) Priorité: 18.09.1991 FR 9111469
(43) Date de publication de la demande: 24.03.1993
(73) Titulaire: ADIR ET COMPAGNIE, F-92415 Courbevoie Cédex (FR)
(72) Inventeur: Regnier, Gilbert, F-92290 Chatenay Malabry (FR); Vilaine, Jean-Paul, F-92290 Chatenay Malabry (FR); Villeneuve, Nicole, F-92500 Rueil Malmaison (FR); Bidouard, Jean-Pierre, F-91380 Chilly Mazarin (FR); Iliou, Jean-Pierre, F-92800 Puteaux (FR); Lenaers, Albert, F-78150 Triel Sur Seine (FR)
(74) Mandataire: Reverbori, Marcelle

(56) Documents cités:
- EP-A- 0 202 580
- WO-A-87/05020
- FR-A- 2 347 359

## Description

La présente invention a pour objet de nouveaux dérivés de la trimétazidine, leur procédé de préparation et les compositions pharmaceutiques les renfermant.

Elle concerne plus particulièrement les dérivés de la trimétazidine de formule générale I : 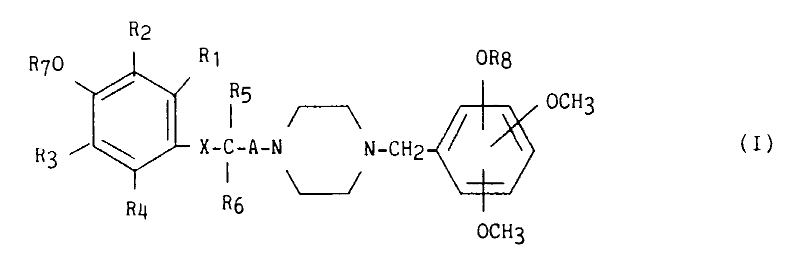 dans laquelle :
- R₁ et R₄, identiques ou différents, représentent chacun un atome d'hydrogène, un radical méthyle ou un radical méthoxy ;
- R₂ et R₃, identiques ou différents, représentent chacun un radical alkyle ou alkoxy contenant chacun de 1 à 6 atomes de carbone en chaine droite ou ramifiée ;
- R₅ et R₆, identiques ou différents, représentent chacun un atome d'hydrogène ou un radical méthyle ;
- R₇ représente un atome d'hydrogène ou un radical acyle de formule : R'₇-CO- dans laquelle R'₇ représente un radical alkyle de 1 à 5 atomes de carbone en chaine droite ou ramifiée, un radical phényle ou phénylméthyle chacun éventuellement substitué, sur le cycle benzénique, par un radical alkyle ou alkoxy ayant chacun de 1 à 5 atomes de carbone en chaîne droite ou ramifiée ;
- R₈ représente un atome d'hydrogène ou un radical alkyle contenant de 1 à 5 atomes de carbone, en chaine droite ou ramifiée ;
- X représente une liaison simple, un atome d'oxygène ou un atome de soufre, et
- A représente une chaine hydrocarbonée droite ou ramifiée contenant de 2 à 6 atomes de carbone éventuellement substituée par un radical hydroxy.

Dans le cas où A comporte un carbone chiral, les dérivés correspondants peuvent exister sous forme d'énantiomères ou de diastéréoisomères qui font également partie de la présente invention.

L'état antérieur de la technique le plus proche de la présente invention est illustré :
- par la demande de brevet européen publiée sous le n° 0202580, laquelle concerne, entre autres, les composés du 3,4-dihydrobenzopyrane dont les produits les plus proches de la présente invention ont pour formule : 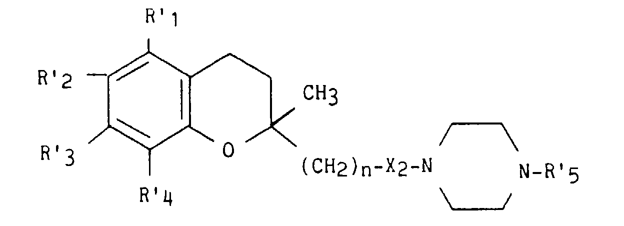 dans laquelle :
   - R′₁ et R′₄ sont hydrogène ou alkyle inférieur,
   - R′₂ est hydrogène, alkoxy inférieur ou alcényloxy inférieur, mais n'est jamais hydroxy
   - R′₃ est hydrogène, alkyl inférieur ou alkoxy inférieur,
   - n est zéro, un ou deux,
   - X₂ peut être entre autres CH₂,
   - R′₅ peut être un benzyl polysubstitué, entre autres, par des radicaux alkoxy inférieur ;
   de tels composés inhibent l'ulcère peptique et ont une activité antitussive et/ou antiexpectorante ; et
- par la demande de brevet PCT, publiée sous le n° WO 87/05020 le 27 août 1987, qui a pour objet des dérivés du 3,4-dihydro-2H-benzopyrane de formule : 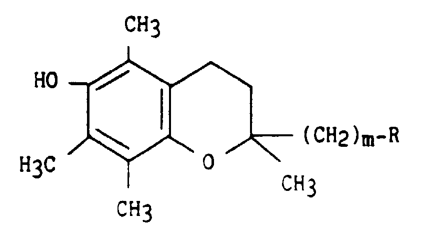 dans laquelle :
   - m représente 1, 2 ou 3 et
   - R représente, entre autres, 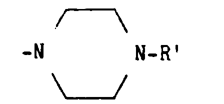 R' étant, entre autres, un radical aralkyle éventuellement substitué ;
      Ces dits dérivés étant utilisables comme médicaments ayant notamment une activité anti-ischémique.

Des modifications de structure ont conduit aux dérivés de formule I de la présente invention, lesquels présentent une activité antihypoxique et anti ischémique particulièrement intéressante.

La présente invention a également pour objet le procédé de préparation des dérivés de formule générale I caractérisé en ce que :
- l'on fait réagir un dérivé de formule générale II : 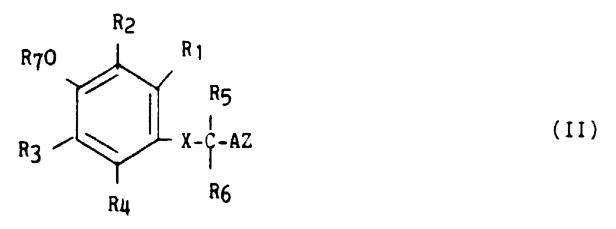 dans laquelle :
   R₁, R₂, R₃, R₄, R₅, R₆, R₇ et A ont les significations précédemment définies,
   et
   - Z représente un atome d'halogène, tel qu'un atome de chlore ou de brome ou un radical tosyloxy,
- avec un dérivé de formule générale III : 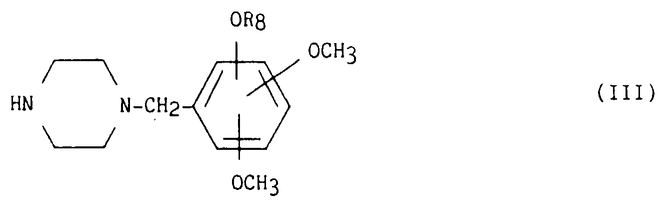
dans laquelle R₈ a la signification précédemment défine, et dans le cas où R₇ représente un radical acyle, le produit de la réaction peut, si on le désire, être hydrolysé pour donner le dérivé hydroxylé correspondant.

Dans certains cas, la réaction des dérivés II et III peut s'effectuer sans solvant, avec un excès de dérivé III qui sert d'accepteur de l'acide formé au cours de la réaction, à une température comprise entre 80 et 180 °C.

Dans la plupart des cas, une telle réaction s'effectue dans un solvant choisi parmi les hydrocarbures aromatiques comme par exemple, le toluène et le xylène, et les solvants aprotiques polaires comme le cyanure de méthyle, le diméthylformamide et le diméthylacétamide, en opérant à une température comprise entre 80 et 130 °C, en présence d'un accepteur de l'acide ZH formé au cours de la réaction -cet accepteur étant soit un carbonate de sodium ou de potassium, soit la triéthylamine, soit un excès du dérivé III.

L'hydrolyse du produit de la réaction, dans le cas où R₇ est un groupe acyle est réalisée par chauffage pendant quelques heures en milieu basique ou en milieu acide dilué, dans un alcool à bas poids moléculaire, selon la nature de R.

Les produits ainsi obtenus sont purifiés par cristallisation de sels au sein de solvants polaires ou plus généralement par chromato-flash sur support de silice (35-70µ) en éluant avec des systèmes appropriés tels que : CH₂Cl₂/CH₃OH, CH₃COOC₂H₅/CH₃OH, CH₃COOC₂H₅/acétone, CH₂Cl₂/CH₃COCC₂H₅.

Les dérivés de formule générale I peuvent être transformés en sels d'addition avec les acides, sels qui font, à ce titre, partie de l'invention. Comme acides utilisables pour la formation de ces sels, on peut citer, par exemple, dans la série minérale les acides chlorhydrique, bromhydrique, nitrique, sulfurique, phosphorique et dans la série organique, les acides acétique, propionique, maléique, fumarique, tartrique, oxalique, benzoïque, méthane sulfonique et iséthionique.

Les dérivés de la présente invention possèdent des propriétés pharmacologiques et thérapeutiques intéressantes. En particulier pour ces dérivés, ont été mises en évidence :

### In vitro

d'une part : leur activité antihypoxique prévenant la dysfonction de coeurs isolés de rats au cours de protocoles d'hypoxie réoxygénation et leur activité relaxante vasculaire, d'autre part : leur capacité à protéger les cellules cardiaques vis à vis d'une nécrose oxydative et les LDL humaines (lipoprotéines de faible densité assurant le transport du cholestérol) vis à vis des modifications oxydatives induites par le cuivre,

### In vivo

leur activité antiischémique au cours de protocoles d'ischémie myocardique induite par sténose coronaire chez le porc.

Ces propriétés permettent l'utilisation des dérivés de la présente invention comme médicaments dans le traitement préventif et curatif des pathologies ischémiques, notamment dans le domaine cardiovasculaire : angine de poitrine, infarctus du myocarde, et les conséquences des cardiopathies ischémiques (trouble du rythme, insuffisance cardiaque) et de la pathologie vasculaire périphérique.

Les dérivés de la présente invention peuvent également être utilisés dans le domaine cérébral, notamment pour le traitement de l'accident vasculaire cérébral et des manifestations déficitaires liées aux troubles circulatoires cérébraux chroniques ; dans le domaine ophtalmologique : notamment pour le traitement des troubles rétiniens d'origine vasculaire ; et dans les manifestations neurosensorielles d'origine ischémique.

Les propriétés antioxydantes de ces produits les rendent en outre intéressants pour la prévention ou le traitement des pathologies dans lesquelles une peroxydation lipidique joue un rôle initiateur et/ou aggravant : telles que, par exemple, outre les cardiopathies ischémiques déjà citées, les lésions vasculaires athéroscléreuses notamment dans le cadre des dyslipidémies, (les modifications oxydatives des LDL apparaissant en effet actuellement constituer un mécanisme important de la formation et de l'extension des lésions vasculaires athéroscléreuses), la reperfusion d'organes y compris transplantés, les pathologies ischémiques, traumatiques ou dégénératives du système nerveux central ou périphérique, les maladies inflammatoires aigües ou chroniques et les maladies auto-immunes.

La présente invention a également pour objet les compositions pharmaceutiques contenant comme principe actif un dérivé de formule générale I ou un de ses sels physiologiquement tolérable, mélangé ou associé à un ou plusieurs excipients pharmaceutiques appropriés.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par la voie orale, rectale ou parentérale.

La posologie peut varier notablement selon l'âge et le poids du patient, la voie d'administration, la nature de la maladie et les traitements associés et s'échelonne de 1 à 200 mg de principe actif, 2 à 3 fois par jour.

Les exemples suivants illustrent l'invention. Les points de fusion sont déterminés au tube capillaire (cap) ou à la platine chauffante de kofler (K).

### Exemple 1

4-(2,3,4-triméthoxybenzyl)-1-[3-(4-hydroxy-2,3,5-triméthylphénoxy)propyl] pipérazine : 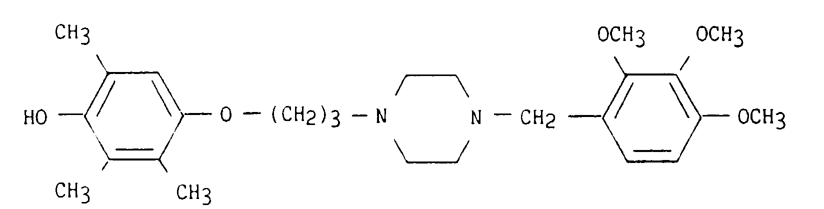 On chauffe, pendant 15 heures à reflux, une solution de 8,4 g de 3-(4-acétoxy-2,3,5-triméthylphénoxy)-1-bromo propane fondant (K) à 51 °C et de 17,6 g de 1-(2,3,4-triméthoxybenzyl) pipérazine dans 200 ml d'acétonitrile, en présence de 0,2 g d'iodure de potassium. Lorsque la réaction est terminée, on évapore le solvant sous pression réduite et purifie le résidu huileux obtenu par chromatoflash sur 600 g de silice en éluant avec CH₂Cl₂-CH₃OH (96-4). On obtient 11 g de 4-(2,3,4-triméthoxybenzyl) -1 -[3 -(4 -acétoxy -2,3,5 -triméthylphénoxy)propyl]pipérazine, fondant (K) à 95 °C.
On hydrolyse ce composé par chauffage à reflux pendant 1 heure dans une solution de 200 ml de méthanol contenant 23,1 ml de soude normale. Après évaporation du solvant, le résidu est repris dans 200 ml d'éther et la solution est lavée à l'eau plusieurs fois. Après évaporation, on obtient 9,6 g d'un produit huileux que l'on transforme en difumarate au sein de l'éthanol. On obtient finalement 12,5 g de difumarate de 4-(2,3,4-triméthoxybenzyl) -1-[3-(4-hydroxy-2,3,5-triméthylphénoxy)propyl]pipérazine, sous forme de cristaux fondant (K) à 200 °C.

### Exemple 2

4-(2,3,4-triméthoxybenzyl)-1-[3-(4-hydroxy-3,5-ditert-butyl phénylthio)-3,3-diméthylpropyl]pipérazine : 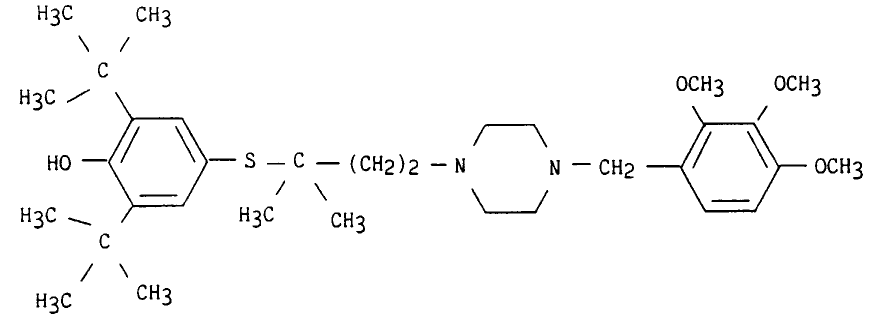 On chauffe pendant 15 heures à reflux, une solution de 9 g de 3-[4-hydroxy-3,5-ditert-butylphénylthio] -3,3-diméthyl-1-tosyloxy propane, fondant (K) à 94 °C, et de 10,07 g de 1-(2,3,4-triméthoxybenzyl)pipérazine, dans 200 ml d'acétonitrile, en présence de 0,2 g d'iodure de potassium.
Lorsque la réaction est terminée, on évapore le solvant sous pression réduite, reprend le résidu huileux par 150 ml de CH₂Cl₂, lave à l'eau et évapore. On obtient un produit huileux que l'on purifie par chromatoflash sur 300 g de silice en éluant avec CH₂Cl₂-acétone (90-10). On recueille finalement 3 g de produit que l'on transforme en difumarate au sein de l'éthanol. On obtient finalement 3 g de difumarate de 4-(2,3,4-triméthoxybenzyl) -1 -[3 -(4 -hydroxy -3,5 -ditert -butylphénylthio) -3,3-diméthylpropyl] pipérazine, sous forme de cristaux fondant (K) à 204 °C.

### Exemples 3 à 22 :

En opérant comme décrit dans l'un et/ou l'autre des exemples ci-dessus, ont été préparés les dérivés suivants :
3) 4-(2,3,4-triméthoxybenzyl)-1-[3-(4-hydroxy-3,5-ditert-butylphénoxy) propyl] pipérazine, P.F. (cap) du dichlorhydrate correspondant : 250-254 °C.
4) 4-(2,3,4-triméthoxybenzyl)-1-[3-(4-hydroxy-3,5 ditert-butylphényl) propyl] pipérazine, P.F. (cap) du dichlorhydrate correspondant : 225-228 °C.
5) 4-(2,3,4-triméthoxybenzyl)-1-[3-(4-hydroxy-3,5-ditert-butylphénoxy)-2-hydroxypropyl]pipérazine, P.F. (K) du dichlorhydrate correspondant : 208 °C
6) 4-(2,3,4-trimétnoxybenzyl)-1-[3-(4-hydroxy-3,5-ditert-butylphénylthio)-2-hydroxypropyl] pipérazine, P.F. (K) du difumarate correspondant : 178 °C.
7) 4-(2,3,4 -triméthoxybenzyl) -1 -[3 -(4 -hydroxy-3,5 -ditert-butylphénylthio) propyl] pipérazine, P.F. (K) du difumarate correspondant : 192 °C.
8) 4-(2,3,4-triméthoxybenzyl)-1-[5-(4-hydroxy-3,5-ditert-butylphénoxy) pentyl] pipérazine, P.F. (K) du difumarate correspondant : 210 °C.
9) 4-(2,3,4-triméthoxybenzyl)-1-[5-(4-hydroxy-3,5-ditert-butylphénylthio) pentyl] pipérazine, P.F. (K) du difumarate correspondant : 190 °C.
10) 4-(2,3,4-triméthoxybenzyl)-1-[5-(4-hydroxy-2,3,5-triméthylphénoxy) pentyl] pipérazine, P.F. (K) du difumarate correspondant : 176 °C.
11) 4-(2,3,4-triméthoxybenzyl)-1-[3-(4-hydroxy-2,3,5-triméthylphénoxy)-2-hydroxypropyl] pipérazine, P.F. (K) du difumarate correspondant : 192 °C.
12) 4-(2,3,4 -triméthoxybenzyl) -1 -[3 -(4 -hydroxy -3,5 -ditert-butylphénylthio)-2,2-diméthylpropyl] pipérazine P.F. (K) du difumarate correspondant : 180 °C.
13) 4-(2,3,4-triméthoxybenzyl)-1-[3-(4-hydroxy-2,3,5-triméthylphénylthio) -2-hydroxypropyl] pipérazine, P.F. (K) du difumarate correspondant 158 °C.
14) 4 (2,3,4-triméthoxybenzyl)-1-[3-(4-hydroxy-2,3,5-triméthylphénylthio) propyl] pipérazine, P.F. (K) du dichlorhydrate 0,7 H₂O correspondant 200 °C.
15) 4 -(3,4,5-triméthoxybenzyl)-1-[3-(4-hydroxy-2,3,5-triméthylphénoxy) propyl] pipérazine.
16) 4-(2,4,6 -triméthoxybenzyl)-1-[3-(4-hydroxy-2,3,5-triméthylphénoxy) propyl] pipérazine.
19) 4-(2-hydroxy-3,4-diméthoxy benzyl)-1-[3-(4-hydroxy-2,3,5-triméthylphénylthio)-2-hydroxypropyl] pipérazine.
20) 4-(2,3,4-triméthoxybenzyl)-1-[3-(4-acétoxy-2,3,5-triméthylphénylthio)-2-hydroxypropyl] pipérazine.
21) (R⁻) 4-(2,3,4-triméthoxybenzyl)-1-[3-(4-hydroxy-2,3,5-triméthylphénylthio)-2-hydroxypropyl] pipérazine, et son bis méthanesulfonate correspondant, dont PF(K) = 164 °C et α²¹_{D}, = -19,9 ° (C=1 %, C₂H₅OH).
22) (S⁺) 4-(2,3,4-triméthoxybenzyl)-1-[3-(4-hydroxy-2,3,5-triméthylphénylthio)-2-hydroxypropyl] pipérazine et son bis méthanesulfonate correspondant, dont PF(K) = 165 °C, et α²¹_{D} = + 21,1 ° (C=1 %, C₂H₅OH).

Certaines des matières premières halogénées ou toxyloxy utilisées pour synthétiser les dérivés I, sont décrites dans la demande de brevet européen publiée sous le n° 0.433.167 A₁, pages 10 et 11. Les autres sont répertoriées ci-après.

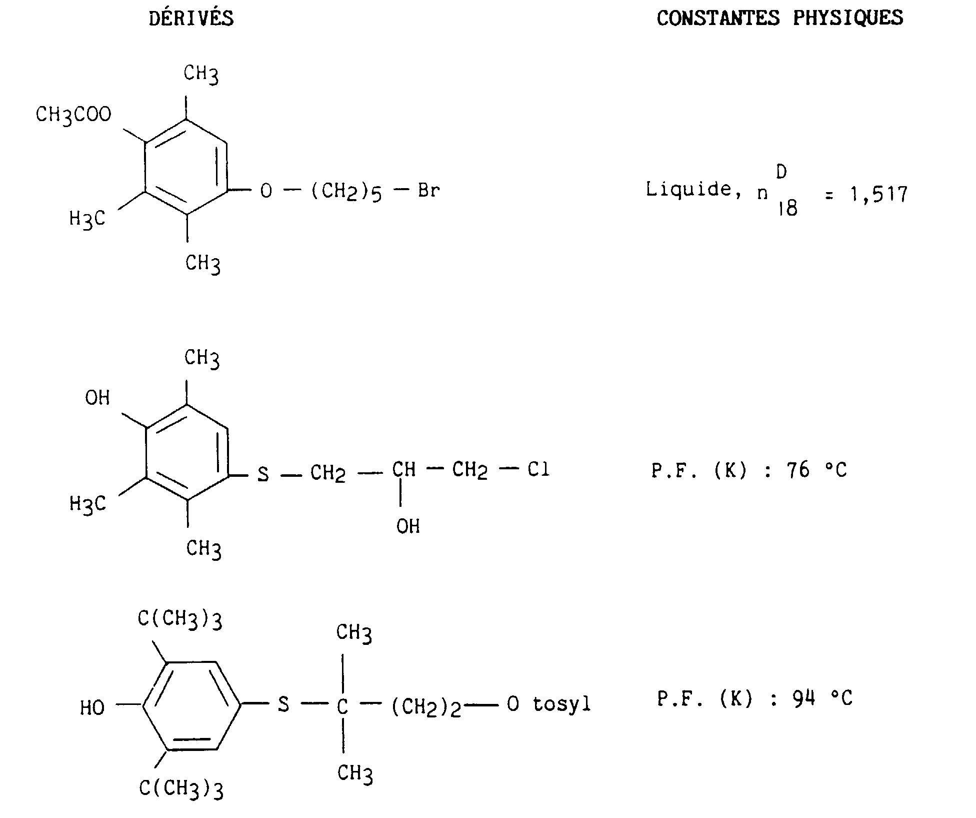

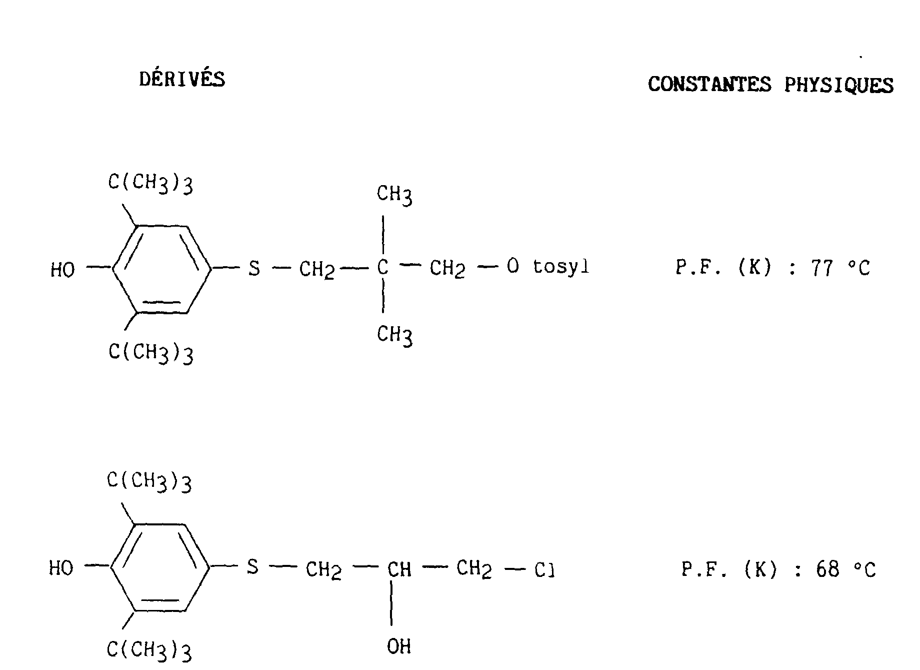

Tous ces dérivés ont été préparés comme mentionné, en pages 10 et 11 de la demande de brevet européen n° 0.433.167 A₁, pour la préparation de dérivés analogues.

### Exemple 23

### ETUDE PHARMACOLOGIQUE

L'activité protectrice cardiaque des produits de la présente invention a été démontrée d'une part in vitro : sur des coeurs isolés de rats soumis à un cycle d'hypoxie réoxygénation ainsi que sur des cellules cardiaques de rats soumises à une nécrose oxydative et d'autre part in vivo : au cours d'épisodes d'ischémie myocardique induite par sténose coronaire chez le porc.
En outre, ont été démontrées pour ces composés in vitro : leur activité relaxante vasculaire sur vaisseaux isolés de rats, et leur activité protectrice des LDL sur des LDL humaines soumises à une modification oxydative induite par le sulfate de cuivre.

### A - ETUDE IN VITRO

### 1. Matériels et méthodes

### a) Hypoxie réoxygénation sur coeur isolé de rat

Les coeurs de rats mâles Wistar (325-375 g - élevage Charles River) anesthésiés par voie intrapéritonéale au pentobarbital sodique (30 mg/kg) sont prélevés après injection d'héparine IV (1 ml/kg) et rapidement perfusés selon la technique de Langendorff à une pression constante de 76 mmHg et stimulés électriquement à 5 Hz. Les contractions isovolumétriques sont enregistrées par l'intermédiaire d'un ballonnet en polyéthylène relié à un capteur de pression (P23-Gould) introduit dans le ventricule gauche et gonflé de manière à obtenir une pression diastolique d'environ 10 mmHg. La solution physiologique utilisée, thermostatée à 37 °C, a la composition suivante (mM) : NaCl 118 ; KCl 4,7 ; KH₂PO₄ 1,2 ; MgCl₂ 1,2 ; CaCl₂ 1,3 ; NaHCO3 25 ; Glucose 8, pH 7,4/95 % O₂ + 5 % CO₂.

Après une période de stabilisation de 15 à 30 minutes, le coeur est soumis à une hypoxie de 60 minutes (réalisée par 95 % N₂ + 5 % CO₂ ; P_{O2}<40 mmHg) suivie par une réoxygénation de 30 minutes ; le produit est mis en incubation 15 minutes avant et pendant la durée de l'hypoxie.

### b) Nécrose oxydative de cellules cardiaques

Les cellules cardiaques de rats-nouveau-nés sont utilisées entre les 5ème et 6ème jours après la mise en culture. La nécrose oxydative est induite par le système enzymatique producteur de radicaux libres hypoxanthine (HX, 1mM) et xanthine-oxydase (X0, 10 mU/ml). La nécrose est évaluée 4 heures après l'adjonction de X0/HX en mesurant spectrophotométriquement l'activité cytosolique α-hydroxy-butyrate deshydrogénase (α-HBDH) libérée dans le surnageant. Les cellules sont traitées avec les molécules testées 16 heures et 1 heure avant le début de l'expérimentation après renouvellement du milieu. En début d'expérience, le traitement est renouvelé une dernière fois.

### c) Etude sur vaisseaux isolés

L'aorte thoracique de rats Wistar (325-375 g) anesthésiés par voie intrapéritonénale au pentobarbital sodique (30 mg/kg) est prélevée et disséquée en anneaux de 3 mm de longueur ; l'endothélium est éliminé par légère abrasion à l'aide d'un morceau de bois. Chaque anneau est accroché à un capteur de tension Statham (UC2-Gould).
La tension initiale appliquée est de 2.5 g. La solution physiologique utilisée, thermostatée à 37 °C, a la composition suivante (mM) : NaCl 112; KCl 5 ; KH₂PO₄ 1 ; MgSO₄ 1,2 ; CaCl₂ 2,5 ; NaHCO₃ 25 ; Glucose 11,5 ; EDTA 0,026.
Après une période de stabilisation de 90 minutes, les anneaux aortiques sont soumis à un milieu hyperpotassique (80 mM KCl et 37 mM NaCl). Quand la contraction est stable, des concentrations cumulatives de produit sont additionnées au milieu toutes les 15 minutes. Les valeurs de relaxations obtenues permettent le calcul de l'IC50.

### d) Modification des LDL par le sulfate de cuivre

Les LDL humaines sont incubées 24 heures en présence de sulfate de cuivre (5.10⁻⁶ M) et en absence ou en présence des composés testés (10⁻⁷ M à 10⁻⁴ M).
Après incubation, la peroxydation des LDL est évaluée par électrophorèse sur gel d'agar et par la formation de l'un des produits de la peroxydation lipidique : le malondialdéhyde, (MDA), (Parthasarathy S., Young S.G., Witztum J.L., Pittman R.C. et Steinberg, D. ; J. Clin. Invest. 77 ; 641-644, 1986).
L'activité des composés testés est évaluée par le calcul des concentrations réduisant de 50 % (IC50) la production de MDA par rapport aux expériences contrôles en absence de produit.

### 2. Résultats

### a) Effets sur le coeur isolé de rat soumis à une hypoxie réoxygénation

Les composés des exemples 1 (3.10⁻⁷ M), 10 (10⁻⁷ M) et 13 (7.10⁻⁷ M) en particulier, réduisent la contracture se développant de 35 à 58 % notamment au bout de 60 minutes d'hypoxie, et de plus de 70 % au terme de 30 minutes de réoxygénation (tableau 1). Ils permettent une meilleure récupération fonctionnelle des coeurs au cours de la réoxygénation supérieure à 70 % comparée à 40 % au cours des expériences contrôles (tableau 2).

### b) Effet sur la nécrose oxydative de cellules cardiaques

Le tableau 3 regroupe les index de nécrose de cellules cardiaques induite par le système hypoxanthine/xanthine-oxydase seul ou en présence de concentrations croissantes des dérivés de l'invention.
Une protection, concentration dépendante, vis à vis de la nécrose est observée. Les composés, notamment des exemples, 1, 3, 9 et 10 s'avèrent protecteurs dès la concentration de 10⁻⁷ M. La plupart des composés réduisent la nécrose de plus de 70 % à la concentration de 10⁻⁵ M.

### c) Effet sur les vaisseaux isolés

Les composés présentés dans le tableau 4 ont une activité relaxante vasculaire vis à vis d'une contraction induite par dépolarisation potassique, leur IC50 se situant entre 2 et 5.10⁻⁶ M.

### d) Effet sur la modification des LDL

Le tableau 5 rassemble les IC50, appréciant le pouvoir inhibiteur de la peroxidation lipidique des LDL humaines induite par le sulfate de cuivre, qui se situent entre 3.10⁻⁸ M et 3.10⁻⁶ M.

**Tableau 1**

| **Effets des composés (I) sur la contracture développée au cours d'une hypoxie réoxygénation de coeurs isolés de rats.** | | | | | |
|---|---|---|---|---|---|
| **COMPOSES** | **CONTRACTURE (mm Hg)** | | | | |
| | **HYPOXIE** | | | **REOXYGENATION** | |
| | 10 min | 30 min | 60 min | 15 min | 30 min |
| **Contrôle** | 23,7±6,8 | 33,7±8,9 | 32,3±7,4 | 20,6±7,8 | 12,3±6,1 |
| **Exemple 1 (3.10**^{**-7**} **M)** | 21,0±5,8 | 22,5±2,5 | 21,0±3,9 | 12,5±5,1 | 2,5+2,5 |
| **Exemple 10 (10**^{**-7**} **M)** | 15,3±3,7 | 16,0±6,4 | 14,7±4,4 | 7,3±5,5 | 2,7±2,7 |
| **Exemple 13 (7.10**^{**-7**} **M)** | 11,0±4,4 | 15,0±6,1 | 13,5±6,3 | 6,0±6,0 | 3,5±3,5 |

**Tableau 2**

| **Effet des composés (I) sur la récupération fonctionnelle de coeurs isolés de rats après hypoxie** | |
|---|---|
| **COMPOSES** | **Pression ventriculaire gauche 30 minutes après réoxygénation (exprimée en % de la valeur initiale avant hypoxie)** |
| **Contrôle** | 41,6±8,8 |
| **Exemple 1 (3.10**^{**-7**}**M)** | 80,5±15 |
| **Exemple 10 (10**^{**-7**}**M)** | 75,9±2,1 |
| **Exemple 13 (7.10**^{**-7**}**M)** | 113,7±18 |

**Tableau 3**

| **Effets des composés (I) sur la nécrose oxydative de cellules cardiaques** | | | | |
|---|---|---|---|---|
| **COMPOSES** | **CONTROLE XO+HX** | **10**^{**-7**}**M XO+HX** | **10**^{**-6**} **M XO+HO** | **10**^{**-5**}**M XO+HX** |
| Exemple 1 | 100,0±12,6 | 70,4±2,5 | 55,4±6,4 | 13,9±5,3 |
| Exemple 3 | 100,0±6,2 | 72,6±5,7 | 16,9±1,1 | 37,6±2,7 |
| Exemple 4 | 100,0±14,7 | 102,9±12,0 | 22,3±6,5 | 10,2±3,1 |
| Exemple 6 | 100,0±2,5 | 99,5±7,5 | 7,7±0,1 | 8,3±1,4 |
| Exemple 7 | 100,0±4,2 | 85,9±1,7 | 19,3±0,9 | 27,9±3,4 |
| Exemple 8 | 100,0±1,1 | 91,4±3,1 | 21,6±4,2 | 27,2±2,0 |
| Exemple 9 | 100,0±1,0 | 53,0±5,5 | 15,2±2,0 | 33,3±6,6 |
| Exemple 10 | 100,0±8,3 | 88,8±0,4 | 18,8±3,7 | 12,6±1,3 |
| Exemple 11 | 100,0±5,1 | 114,0±5,2 | 70,9±8,3 | 18,0±2,9 |
| Exemple 13 | 100,0±6,1 | 100,1±4,8 | 64,7±0,4 | 15,1±0,6 |

**Tableau 4**

| **Effet relaxant vasculaire des composés (I) sur l'aorte de rat contractée par le potassium (80 mM)** | |
|---|---|
| **COMPOSES** | **IC 50 (M)** |
| Exemple 1 | 5,0.10⁻⁶ |
| Exemple 6 | 2,7.10⁻⁶ |
| Exemple 10 | 2,0.10⁻⁶ |
| Exemple 13 | 4,3.10⁻⁶ |

**Tableau 5**

| **Effet inhibiteur des composés (I) sur la modification des LDL humaines induite par le cuivre** | |
|---|---|
| **COMPOSES** | **IC 50 (M)** |
| Exemple 1 | 9.10⁻⁸ |
| Exemple 2 | 3.10⁻⁶ |
| Exemple 3 | 5.10⁻⁷ |
| Exemple 4 | 2.10⁻⁶ |
| Exemple 5 | 9.10⁻⁷ |
| Exemple 6 | 3.10⁻⁷ |
| Exemple 7 | 6.10⁻⁷ |
| Exemple 8 | 10⁻⁶ |
| Exemple 9 | 8.10⁻⁸ |
| Exemple 10 | 3.10⁻⁸ |
| Exemple 11 | 6.10⁻⁷ |
| Exemple 12 | 8.10⁻⁷ |
| Exemple 13 | 7.10⁻⁸ |

### B - ETUDE IN VIVO

### 1. Matériels et méthodes

L'étude est réalisée sur des porcs "Large White" des deux sexes agés de 3 mois et pesant 21 à 26 kg.
Les animaux sont tranquilisés au midazolam (1 mg/kg IM), puis anesthésiés au thiopental sodique (8 mg/kg IV). L'anesthésie est maintenue par une perfusion de 6 à 8 mg/kg/h.
Ils sont immédiatement intubés et ventilés avec un mélange air + O₂.
Une thoracotomie en "T" est réalisée par section longitudinale du sternum et incision entre la 4ème et la 5ème côte.
Le coeur est suspendu dans un berceau péricardique réalisé par section et fixation du péricarde en quatre points aux muscles thoraciques.

Une bague de débit électromagnétique est placée autour de la branche interventriculaire antérieure de la coronaire gauche, un ballonnet pneumatique est placé immédiatement en aval de cette bague. Cet ensemble étant destiné à la réalisation de sténose coronaire sous contrôle du débit.
Des cristaux piezo-électriques reliés à un sonomicromètre Triton sont implantés dans le sous endocarde de la paroi ventriculaire gauche selon un plan circonférentiel perpendiculaire à l'axe cardiaque. Ces cristaux sont destinés à l'enregistrement de la contractilité régionale myocardique et d'ECG endocardiques dans la zone irriguée par l'artère coronaire sténosée. Dans ces zones sont également enregistrés des ECG épicardiques par l'intermédiaire d'électrodes fixées sur le myocarde.

### 2. Protocole expérimental

L'ischémie myocardique est réalisée par inflation du ballonet induisant une réduction de débit de 50 à 60 %.
Deux sténoses identiques de 3 minutes aux effets reproductibles et réversibles sont réalisées, séparées par un intervalle de récupération de 55 minutes.
Le traitement est administré par voie veineuse 10 minutes avant la sténose en perfusion de 5 minutes :
. perfusion de solvant avant la première sténose
. perfusion du produit ou solvant avant la deuxième sténose.

### 3. Paramètres mesurés

. La contractilité myocardique régionale est appréciée par la fraction de raccourcissement systolique des longueurs segmentaires comprises entre les cristaux piézo-électriques.
. L'hypokinésie en zone ischémique est exprimé en pourcentage de variation par rapport à la contractilité mesurée en période contrôle.
. La modification des ECG épicardique et endocardique en zone ischémique consiste en une surélévation du segment S.T. mésurée en millivolt (mV).

### 4. Résultats

Aucune différence n'est observée entre les effets des deux sténoses coronaires provoquées dans le groupe contrôle sur les électrocardiogrammes, sur la contractilité régionale, une dégradation supérieure est même observée lors de la seconde sténose coronaire.
Par contre, le composé de l'exemple 10 en particulier, administré par voie intraveineuse à la dose de 1 mg/kg avant la seconde sténose coronaire réduit de 45 % la surélévation des segments ST des électrocardiogrammes tant épicardique qu'endocardique par rapport à la première sténose coronaire, et provoque une amélioration de 50 % de la contractilité régionale en zone ischémique par rapport à l'effet de la première sténose coronaire. (cf tableau 6 et 7).

**Tableau 6**

| **Effet du composé de l'exemple 10 sur les modifications des ECG épicardique et endocardique au cours d'une ischémie myocardique chez le porc** | | | | |
|---|---|---|---|---|
| **COMPOSE** | **Surélévation ST (mV) ECG épicardique** | | **Surélévation ST (mV) ECG endocardique** | |
| | **ST1** | **ST2** | **ST1** | **ST2** |
| **Contrôle** | 3,87±0,98 | 3,16±1,2 | 2,28±0,48 | 2,4±0,51 |
| **Exemple 10 (1 mg/kg/IV)** | 4,6±1,0 | 2,5±0,6 | 3,4±0,4 | 1,9±0,5 |

**Tableau 7**

| **Effet du composé de l'exemple 10 sur la contractilité régionale myocardique de zones ischémiques chez le porc** | | |
|---|---|---|
| **COMPOSE** | **% de contractilité myocardique résiduelle** | |
| | **ST1** | **ST2** |
| **Contôle** | 33,2±2,5 | 25,2±3,0 |
| **Exemple 10 (1 mg/kg/IV)** | 20,4±3,3 | 29,4±2,6 |

### C - CONCLUSION

Les résultats rapportés démontrent que les composés de l'invention ont une activité protectrice du tissu cardiaque in vitro vis à vis de stress hypoxique ou oxydatif, et in vivo vis à vis d'un épisode ischémique, ainsi que des activités antispastiques vasculaires et protectrices vis à vis des modifications oxydatives des LDL impliquées dans l'athérogénèse.

## Revendications (Revendications pour l'(les) Etat(s) contractant(s) suivant(s): AT, BE, CH, DE, DK, FR, GB, IE, IT, LI, LU, NL, PT, SE)

1. les dérivés de la trimétazidine de formule générale I : 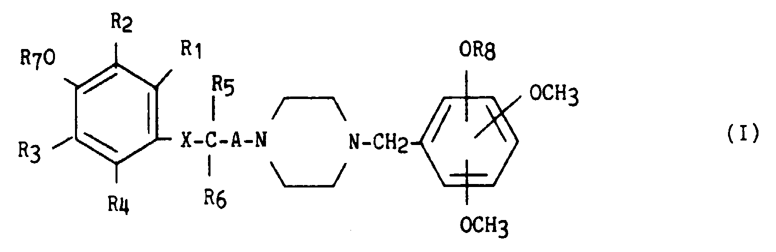 dans laquelle :
- R₁ et R₄, identiques ou différents, représentent chacun un atome d'hydrogène, un radical méthyle ou un radical méthoxy ;
- R₂ et R₃, identiques ou différents, représentent chacun un radical alkyle ou alkoxy contenant chacun de 1 à 6 atomes de carbone en chaîne droite ou ramifiée ;
- R₅ et R₆, identiques ou différents, représentent chacun un atome d'hydrogène ou un radical méthyle ;
- R₇ représente un atome d'hydrogène ou un radical acyle de formule : R'₇-CO- dans laquelle R'₇ represente un radical alkyle de 1 à 5 atomes de carbone en chaîne droite ou ramifiée, un radical phényle ou phénylméthyle chacun éventuellement substitué, sur le cycle benzénique, par un radical alkyle ou alkoxy ayant chacun de 1 à 5 atomes de carbone en chaîne droite ou ramifiée ;
- R₈ représente un atome d'hydrogène ou un radical alkyle contenant de 1 à 5 atomes de carbone, en chaîne droite ou ramifiée ;
- X représente une liaison simple, un atome d'oxygène ou un atome de soufre, et
- A représente une chaine hydrocarbonée droite ou ramifiée contenant de 2 à 6 atomes de carbone éventuellement substituée par un radical hydroxy ; et
lorsque A comporte un carbone chiral, les énantiomères et diastéréoisomères correspondants.

2. Les sels physiologiquement tolérables des dérivés de la revendication 1 avec les acides appropriés.

3. Le 4-(2,3,4-triméthoxybenzyl)-1-[3-(4-hydroxy-2,3,5-triméthylphénoxy) propyl]-pipérazine et son difumarate.

4. Le 4-(2,3,4-triméthoxybenzyl)-1-[5-(4-hydroxy-2,3,5-triméthylphénoxy) pentyl]pipérazine et son difumarate.

5. Le 4-(2,3,4-triméthoxybenzyl)-1-[3-(4-hydroxy-2,3,5-triméthylphénylthio]-2-hydroxypropyl] pipérazine et son difumarate.

6. Le procédé de préparation des dérivés de la revendication 1, caractérisé en ce que :
- l'on fait réagir un dérivé de formule générale II : 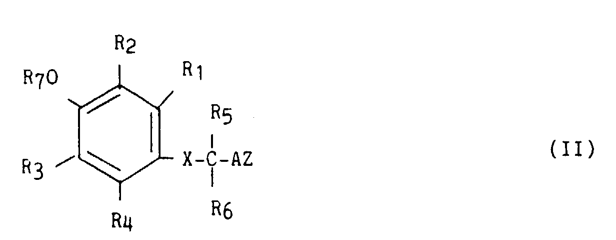 dans laquelle :
R₁, R₂, R₃, R₄, R₅, R₆, R₇ et A ont les significations définies dans la revendication 1,
et
- Z représente un atome d'halogène ou un radical tosyloxy,
- avec un dérivé de formule générale III : 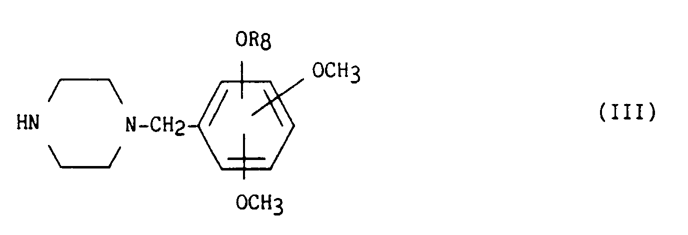 dans laquelle R₈ a la signification définie dans la revendication 1, et dans le cas où R₇ représente un radical acyle, le produit de la réaction peut, si on le désire, être hydrolysé pour donner le dérivé hydroxylé correspondant.

7. Les compositions pharmaceutiques contenant comme principe actif un dérivé selon l'une quelconque des revendications 1 à 5 avec un ou plusieurs excipients pharmaceutiques appropriés.

8. Les compositions pharmaceutiques selon la revendication 7 présentées sous une forme convenant notamment dans le traitement des pathologies ischémiques et de la pathologie vasculaire périphérique.

## Revendications (Revendications pour l'(les) Etat(s) contractant(s) suivant(s): ES, GR)

1. Le procédé de préparation des dérivés de la trimétazidine de formule générale I: 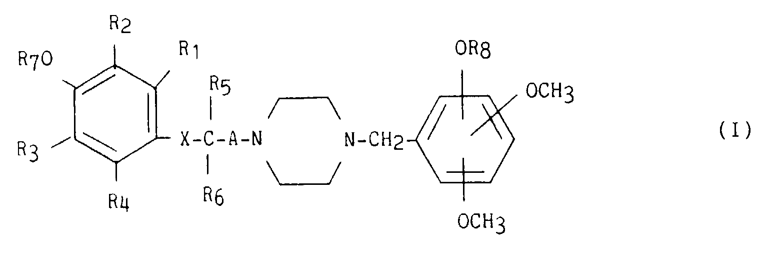 dans laquelle :
- R₁ et R₄, identiques ou différents, représentent chacun un atome d'hydrogène, un radical méthyle ou un radical méthoxy ;
- R₂ et R₃, identiques ou différents, représentent chacun un radical alkyle ou alkoxy contenant chacun de 1 à 6 atomes de carbone en chaine droite ou ramifiée ;
- R₅ et R₆, identiques ou différents, représentent chacun un atome d'hydrogène ou un radical méthyle ;
- R₇ représente un atome d'hydrogène ou un radical acyle de formule : R'₇-CO- dans laquelle R'₇ represente un radical alkyle de 1 à 5 atomes de carbone en chaine droite ou ramifiée, un radical phényle ou phénylméthyle chacun éventuellement substitué, sur le cycle benzénique, par un radical alkyle ou alkoxy ayant chacun de 1 à 5 atomes de carbone en chaine droite ou ramifiée ;
- R₈ représente un atome d'hydrogène ou un radical alkyle contenant de 1 à 5 atomes de carbone, en chaine droite ou ramifiée ;
- X représente une liaison simple, un atome d'oxygène ou un atome de soufre, et
- A représente une chaine hydrocarbonée droite ou ramifiée contenant de 2 à 6 atomes de carbone éventuellement substituée par un radical hydroxy ; et
lorsque A comporte un carbone chiral, des énantiomères et diastéréoisomères correspondants,
ainsi que leurs sels physiologiquement tolérables avec les acides appropriés,
caractérisé en ce que :
- l'on fait réagir un dérivé de formule générale II : 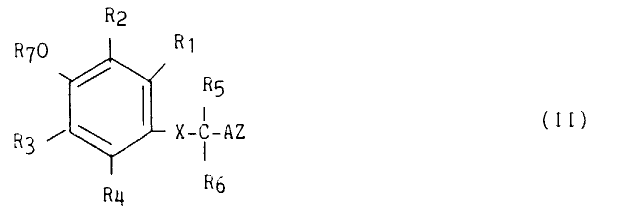 dans laquelle :
R₁, R₂, R₃, R₄, R₅, R_{6,} R₇ et A ont les significations précédemment définies,
et
- Z représente un atome d'halogène ou un radical tosyloxy,
- avec un dérivé de formule générale III : 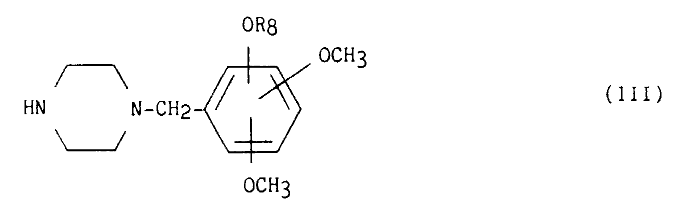 dans laquelle R₈ a la signification précédemment définie, et
dans le cas où R₇ représente un radical acyle, le produit de la réaction peut, si on le désire, être hydrolysé pour donner le dérivé hydroxylé correspondant,
- et si on le désire, on traite les dérivés ainsi obtenus avec des acides appropriés pour donner les sels d'addition acides correspondants.

## Patentansprüche (Patentansprüche für folgende(n) Vertragsstaat(en): AT, BE, CH, DE, DK, FR, GB, IE, IT, LI, LU, NL, PT, SE)

1. Trimetazidin-Derivate der allgemeinen Formel I: 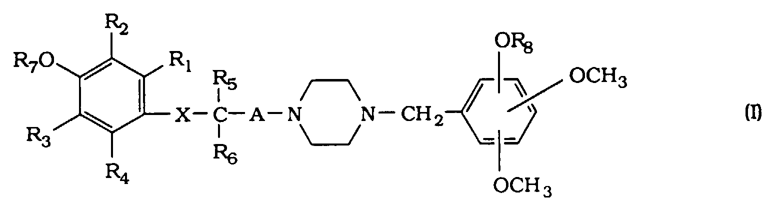 in der:
- R₁ und R₄, die gleichartig oder verschieden sein können, jeweils ein Wasserstoffatom, eine Methylgruppe oder eine Methoxygruppe;
- R₂ und R₃, die gleichartig oder verschieden sein können, jeweils eine Alkyl- oder Alkoxygruppe mit jeweils 1 bis 6 Kohlenstoffatomen in gerader oder verzweigter Kette;
- R₅ und R₆, die gleichartig oder verschieden sein können, jeweils ein Wasserstoffatom oder eine Methylgruppe;
- R₇ ein Wasserstoffatom oder eine Acylgruppe der Formel: R'₇-CO-, in der R'₇ eine Alkylgruppe mit 1 bis 5 Kohlenstoffatomen in gerader oder verzweigter Kette, eine Phenyl- oder Phenylmethylgruppe, die gegebenenfalls am Benzolkern durch eine Alkyl- oderAlkoxygruppe mit jeweils 1 bis 5 Kohlenstoffatomen in gerader oder verzweigter Kette substituiert ist, darstellt;
- R₈ ein Wasserstoffatom oder eine Alkylgruppe mit 1 bis 5 Kohlenstoffatomen in gerader oder verzweigter Kette;
- X eine Einfachbindung, ein Sauerstoffatom oder ein Schwefelatom; und
- A eine geradkettige oder verzweigte Kohlenwasserstoffkette mit 2 bis 6 Kohlenstoffatomen, die gegebenenfalls durch eine Hydroxylgruppe substituiert ist, bedeuten; und
wenn A ein chirales Kohlenstoffatom trägt, die entsprechenden Enantiomere und Diastereoisomere.

2. Die physiologisch verträglichen Salze der Derivate nach Anspruch 1 mit geeigneten Säuren.

3. 4-(2,3,4-Trimethoxybenzyl)-1-[3-(4-hydroxy-2,3,5-trimethylphenoxy)propyl]-piperazin und dessen Difumarat.

4. 4-(2,3,4-Trimethoxybenzyl)- 1-[5-(4-hydroxy-2,3,5-trimethylphenoxy)pentyl]-piperazin und dessen Difumarat.

5. 4-(2,3,4-Trimethoxybenzyl)-1-[3-(4-hydroxy-2,3,5-trimethylphenylthio)-2-hydroxypropyl]-piperazin und dessen Difumarat.

6. Verfahren zur Herstellung der Derivate nach Anspruch 1, **dadurch gekennzeichnet,** daß man:
- ein Derivat der allgemeinen Formel II: 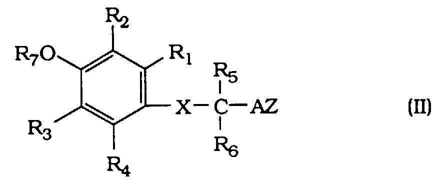 in der:
R₁, R₂, R₃, R₄, R₅, R₆, R₇ und A die in Anspruch 1 angegebenen Bedeutungen besitzen,
und
- Z ein Halogenatom oder eine Tosyloxygruppe darstellt,
- mit einem Derivat der allgemeinen Formel III: 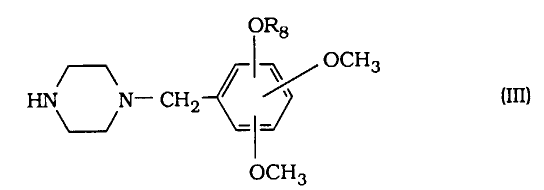 in der R₈ die in Anspruch 1 angegebenen Bedeutungen besitzt, umsetzt und, wenn R₇ eine Acylgruppe darstellt, das Reaktionsprodukt gewünschtenfalls hydrolysiert zur Bildung des entsprechenden Hydroxyderivats.

7. Pharmazeutische Zubereitungen, enthaltend als Wirkstoff ein Derivat nach irgendeinem der Ansprüche 1 bis 5 zusammen mit einem oder mehreren geeigneten pharmazeutischen Trägermaterialien.

8. Pharmazeutische Zubereitungen nach Anspruch 7 in einer Form, die insbesondere bei der Behandlung von ischämischen pathologischen Zuständen und pathologischen Zuständen der peripheren Gefäße geeignet sind.

## Patentansprüche (Patentansprüche für folgende(n) Vertragsstaat(en): ES, GR)

1. Verfahren zur Herstellung von Trimetazidin-Derivaten der allgemeinen Formel I: 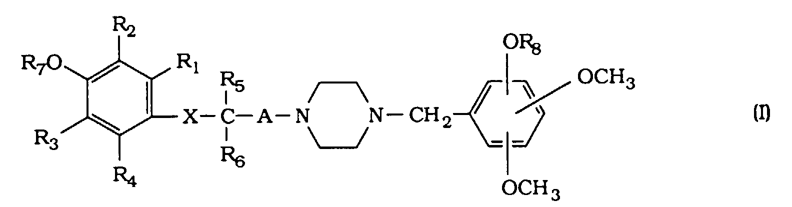 in der:
- R₁ und R₄, die gleichartig oder verschieden sein können, jeweils ein Wasserstoffatom, eine Methylgruppe oder eine Methoxygruppe;
- R₂ und R₃, die gleichartig oder verschieden sein können, jeweils eine Alkyl- oder Alkoxygruppe mit jeweils 1 bis 6 Kohlenstoffatomen in gerader oder verzweigter Kette;
- R₅ und R₆, die gleichartig oder verschieden sein können, jeweils ein Wasserstoffatom oder eine Methylgruppe;
- R₇ ein Wasserstoffatom oder eine Acylgruppe der Formel: R'₇-CO-, in der R'₇ eine Alkylgruppe mit 1 bis 5 Kohlenstoffatomen in gerader oder verzweigter Kette, eine Phenyl- oder Phenylmethylgruppe, die gegebenenfalls am Benzolkern durch eine Alkyl- oderAlkoxygruppe mit jeweils 1 bis 5 Kohlenstoffatomen in gerader oder verzweigter Kette substituiert ist, darstellt;
- R₈ ein Wasserstoffatom oder eine Alkylgruppe mit 1 bis 5 Kohlenstoffatomen in gerader oder verzweigter Kette;
- X eine Einfachbindung, ein Sauerstoffatom oder ein Schwefelatom; und
- A eine geradkettige oder verzweigte Kohlenwasserstoffkette mit 2 bis 6 Kohlenstoffatomen, die gegebenenfalls durch eine Hydroxylgruppe substituiert ist, bedeuten; und
wenn A ein chirales Kohlenstoffatom aufweist, von den entsprechenden Enantiomeren und Diastereoisomeren sowie von deren physiologisch verträglichen Salzen mit geeigneten Säuren, **dadurch gekennzeichnet,** daß man:
- ein Derivat der allgemeinen Formel II: 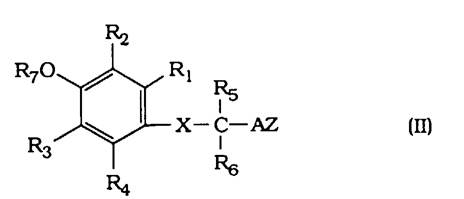 in der:
R₁, R₂, R₃, R₄, R₅, R₆, R₇ und A die oben angegebenen Bedeutungen besitzen,
und
- Z ein Halogenatom oder eine Tosyloxygruppe darstellt,
- mit einem Derivat der allgemeinen Formel III: 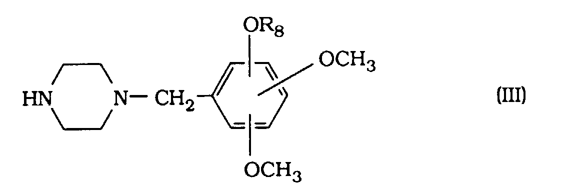 in der R₈ die oben angegebenen Bedeutungen besitzt, umsetzt und, wenn R₇ eine Acylgruppe darstellt, das Reaktionsprodukt gewünschtenfalls hydrolysiert zur Bildung des entsprechenden Hydroxyderivats,
- und gewünschtenfalls die in dieser Weise erhaltenen Derivate mit geeigneten Säuren behandelt zur Bildung der entsprechenden Säureadditionssalze.

## Claims (Claims for the following Contracting State(s): AT, BE, CH, DE, DK, FR, GB, IE, IT, LI, LU, NL, PT, SE)

1. Trimetazidine compounds of the general formula I : 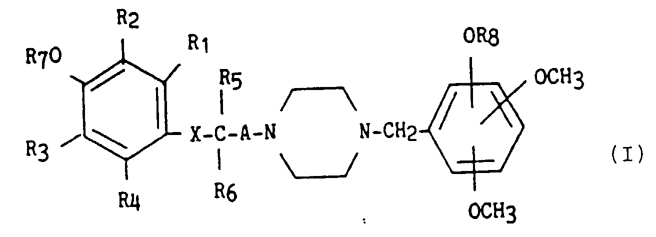 wherein :
- R₁ and R₄, which are the same or different, each represents a hydrogen atom, a methyl radical or a methoxy radical;
- R₂ and R₃, which are the same or different, each represents a straight-chain or branched alkyl or alkoxy radical each having from 1 to 6 carbon atoms;
- R₅ and R₆, which are the same or different, each represents a hydrogen atom or a methyl radical;
- R₇ represents a hydrogen atom or an acyl radical of formula R'₇-CO- wherein R'₇ represents a straight-chain or branched alkyl radical having from 1 to 5 carbon atoms, or a phenyl or phenylmethyl radical each of which is optionally substituted in the benzene ring by a straight-chain or branched alkyl or alkoxy radical each having from 1 to 5 carbon atoms;
- R₈ represents a hydrogen atom or a straight-chain or branched alkyl radical containing from 1 to 5 carbon atoms;
- X represents a single bond, an oxygen atom or a sulphur atom, and
- A represents a straight-chain or branched hydrocarbon chain containing from 2 to 6 carbon atoms that is optionally substituted by a hydroxy radical; and
when A contains a chiral carbon atom, the corresponding enantiomers and diastereoisomers.

2. The physiologically tolerable salts of the compounds of claim 1 with appropriate acids.

3. 4-(2,3,4-trimethoxybenzyl)-1-[3-(4-hydroxy-2,3,5-trimethylphenoxy)propyl]piperazine and its difumarate.

4. 4-(2,3,4-trimethoxybenzyl)-1-[5-(4-hydroxy-2,3,5-trimethylphenoxy)pentyl]piperazine and its difumarate.

5. 4-(2,3,4-trimethoxybenzyl)-1-[3-(4-hydroxy-2,3,5-trimethylphenylthio)-2-hydroxypropyl]piperazine and its difumarate.

6. A process for the preparation of compounds of claim 1, characterised in that :
- a compound of the general formula II : 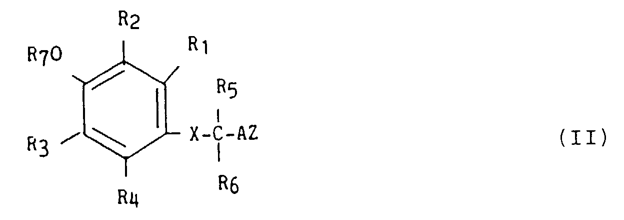 wherein :
R₁, R₂, R₃, R₄, R₅, R₆, R₇ and A have the meanings given in claim 1,
and
- Z represents a halogen atom or a tosyloxy radical,
- is reacted with a compound of the general formula III : 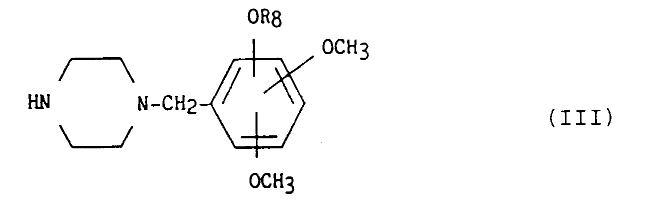 wherein R₈ has the meaning given in claim 1 and, when R₇ represents an acyl radical, the product of the reaction may, if desired, be hydrolysed to yield the corresponding hydroxylated compound.

7. Pharmaceutical compositions comprising as active ingredient a compound according to any one of claims 1 to 5 together with one or more appropriate pharmaceutical excipients.

8. Pharmaceutical compositions according to claim 7 presented in a form suitable in particular for the treatment of ischaemic pathologies and peripheral vascular pathology.

## Claims (Claims for the following Contracting State(s): ES, GR)

1. A process for the preparation of trimetazidine compounds of the general formula I : 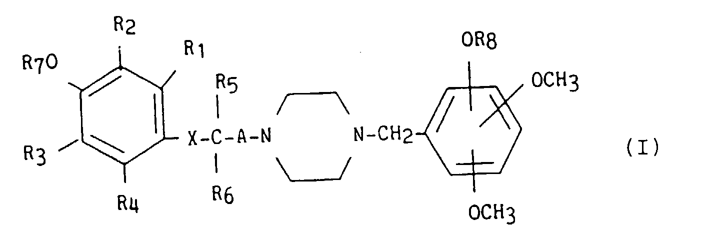 wherein :
- R₁ and R₄, which are the same or different, each represents a hydrogen atom, a methyl radical or a methoxy radical;
- R₂ and R₃, which are the same or different, each represents a straight-chain or branched alkyl or alkoxy radical each having from 1 to 6 carbon atoms;
- R₅ and R₆, which are the same or different, each represents a hydrogen atom or a methyl radical;
- R₇ represents a hydrogen atom or an acyl radical of formula R'₇-CO- wherein R'₇ represents a straight-chain or branched alkyl radical having from 1 to 5 carbon atoms, or a phenyl or phenylmethyl radical each of which is optionally substituted in the benzene ring by a straight-chain or branched alkyl or alkoxy radical each having from 1 to 5 carbon atoms;
- R₈ represents a hydrogen atom or a straight-chain or branched alkyl radical containing from 1 to 5 carbon atoms;
- X represents a single bond, an oxygen atom or a sulphur atom, and
- A represents a straight-chain or branched hydrocarbon chain containing from 2 to 6 carbon atoms that is optionally substituted by a hydroxy radical; and
when A contains a chiral carbon atom, corresponding enantiomers and diastereoisomers,
and also the physiologically tolerable salts thereof with appropriate acids,
characterised in that :
- a compound of the general formula II : 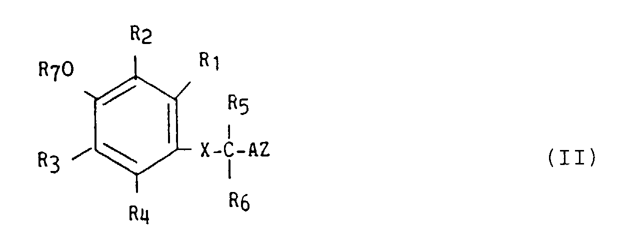 wherein :
R₁, R₂, R₃, R₄, R₅, R₆, R₇ and A are as defined above,
and
- Z represents a halogen atom or a tosyloxy radical,
- is reacted with a compound of the general formula III : 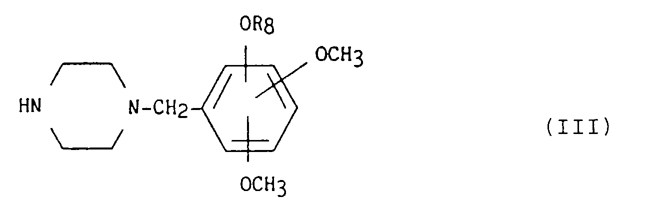 wherein R₈ is as defined above, and, when R₇ represents an acyl radical, the product of the reaction may, if desired, be hydrolysed to yield the corresponding hydroxylated compound,
- and, if desired, the compounds so obtained are treated with appropriate acids to yield the corresponding acid addition salts.
